(19) Europäisches Patentamt
European Patent Office
Office européen des brevets

(11) **EP 1 926 445 B1**

(12) **EUROPÄISCHE PATENTSCHRIFT**

(45) Veröffentlichungstag und Bekanntmachung des Hinweises auf die Patenterteilung:
**22.05.2013 Patentblatt 2013/21**

(21) Anmeldenummer: **06805774.4**

(22) Anmeldetag: **20.09.2006**

(51) Int Cl.:
*A61B 17/80* (2006.01)    *A61B 17/86* (2006.01)

(86) Internationale Anmeldenummer:
**PCT/EP2006/009137**

(87) Internationale Veröffentlichungsnummer:
**WO 2007/036318 (05.04.2007 Gazette 2007/14)**

(54) **OSTEOSYNTHESEHILFSMITTEL**

OSTEOSYNTHESIS AID

AUXILIAIRE D'OSTEOSYNTHESE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **23.09.2005 DE 102005045739**

(43) Veröffentlichungstag der Anmeldung:
**04.06.2008 Patentblatt 2008/23**

(73) Patentinhaber: **Universitätsklinikum Freiburg**
**79106 Freiburg (DE)**

(72) Erfinder:
• **PETERS, Christian**
**79100 Freiburg (DE)**
• **MANOLI, Yiannos**
**79104 Freiburg (DE)**
• **GUTWALD, Ralf**
**79258 Hartheim (DE)**
• **SCHMELZEISEN, Rainer**
**79102 Freiburg (DE)**

(74) Vertreter: **Huwer, Andreas et al**
**Schwaighofstrasse 1**
**79100 Freiburg (DE)**

(56) Entgegenhaltungen:
WO-A-01/49173      WO-A2-2005/074821
DE-A1- 4 132 021   DE-A1- 10 342 823
US-A- 6 034 296    US-A1- 2004 204 647

• PUERS R ET AL: "AN IMPLANTABLE SYSTEM FOR DETECTING LOOSENING OF A HIP PROSTHESIS" INTERNATIONAL SYMPOSIUM ON BIOTELEMETRY, 9. Mai 1999 (1999-05-09), Seiten 652-660, XP008034489

**Beschreibung**

Die Erfindung betrifft ein Osteosynthesehilfsmittel nach dem Oberbegriff von Anspruch 1.

Ein derartiges Osteosynthesehilfsmittel ist aus US 2004/0204647 A1 bekannt. Es hat ein am Knochen anbringbares, als Osteosyntheseplatte ausgestaltetes Verstärkungselement, Knochenschrauben zum Verbinden des Verstärkungselements mit dem Knochen und eine mit einer Auswerte- und/oder Anzeigeeinrichtung verbundene Messeinrichtung, die mindestens einen Kraftsensor aufweist, der als flächiges, quer zu seiner Erstreckungsebene druckempfindliches Element ausgebildet ist. Bei diesem Osteosynthesehilfsmittel weist der Kraftsensor ein Glasfasemetz auf, das in das Verstärkungselement eingebettet ist. Durch das Glasfasernetz wird optische Strahlung geleitet und es werden Veränderungen der Eigenschaften der optischen Strahlung, die bei mechanischen Spannungen in dem Verstärkungselement auftreten, gemessen. Nachteilig ist bei diesem Osteosynthesehilfsmittel, dass Fehler bei der Frakturheilung mit Hilfe der Messeinrichtung nur begrenzt festgestellt werden können.

Aus WO 2005/074821 A2 ist ferner ein Osteosynthesehilfsmittel bekannt, das ein Verstärkungselement aufweist welches mittels Knochenschrauben mit einem Knochen verbunden ist. An dem Verstärkungselement ist ein Bauteil angebracht, das ein Grundbauteil hat, an dem ein Druck- oder Dehnungssensor und eine Telemetrieeinrichtung angeordnet sind. Ein Teil der zur Montage des Verstärkungselements nutzbaren Öffnungen des Verstärkungselements werden nicht zum Befestigen mittels der Knochenschrauben genutzt. In eine dieser nicht genutzten Öffnungen ist das den Sensor aufweisende Bauteil eingeführt.

Ein Osteosynthesehilfsmittel, das Knochenschrauben zum Befestigen einer Osteosynthese-Platte an einem Kieferknochen aufweist, ist ferner aus DE 90 04 717 U1 bekannt. Mit der Osfeasynthese-Platte wird ein zu behandelnder Frakturspalt des Kieferknochens überbrückt. Beidseits des Frakturspalts hat die Osteosynthese-Platte jeweils zwei kreisförmige Öffnungen und einer längliche Öffnung, welche die mit dem Kieferknochen verschraubten Knochenschrauben In Gebrauchsstellung durchsetzen. Durch die so geblldete, fest mit dem Kieferknochen verbundene Brücke werden die beidseits der Knochenstelle befindlichen Bereiche des Kieferknochens in einer festen Lage relativ zueinander fixiert, Ziel der Behandlung ist einerseits die Stabilisierung der Knochenwunde am Frakturspalt und andererseits eine möglichst frühzeitig aktive und schmerzfreie Mobilisierung der frakturnahen Muskulatur und Gelenke. Dadurch soll dem Auftreten der so genannten Frakturkrankheit vorgebeugt werden, die bei einer gestörten Knochenheilung im Bereich des Bruchs auftritt. Die Frakturkrankheit ist unter anderem gekennzeichnet durch chronische Ödembildung, Atrophie der Weichteile, Knochenabbau, Arthrosen und Gelenkversteifungen. Die Frakturkrankheit kann auch zu Okklusionsstörungen führen. In der Praxis zeigen sich bei einem Teil der behandelten Patienten postoperative Komplikationen. In der Regel sind dies Infektionen des Bruchspalts, die durch eine Instabilität des implantats hervorgerufen und unterhalten werden. Damit wird für die betreffenden Patienten eine zweite Operation zwingend notwendig, um einerseits die infizierten Knochenbereiche zu entfernen und andererseits eine wesentlich größere Osteosyntheseplatte einzusetzen, die insgesamt eine größere Stabilität aufweist als die erste Osteosyntheseplatte, bei der die Komplikationen aufgetreten sind. Um die größere Osteosyntheseplatte an dem Kieferknochen zu fixieren, ist ein erheblich größerer Zugangsweg erforderlich als bei der ersten Osteosyntheseplatte. Teilweise werden auch so genannte Osteoplastiken (Knochentransplantationen) erforderlich, um den geschädigten Knochen zu ersetzten. Die transplantierten Knochenstücke werden ebenfalls wieder mit Schrauben an dem Kieferknochen fixiert. Dies bedeutet für den Patienten eine erhebliche Mehrbelastung, die unter Umständen auch zu einer bleibenden invalidität führt. Zusätzlich entstehen Folgekosten (Zweitoperation, Hospitalisierung, Arbeitsausfall, Invalidität usw.).

Trotz modernster diagnostischer Einrichtungen bestehen bisher nur wenige Möglichkeiten, die Frakturhelung im Verlauf zu überprüfen. Sie kann entweder klinisch oder mit Hilfe von radiologischer Diagnostik, wie z.B. Röntgen oder Computertomographie (CT), untersucht werden. Ein Nachteil der klinischen Beurteilung ist, dass sich die ersten Symptome einer Komplikation (Beweglichkeit der Knochensegmente, Infektionen usw.) erst zeigen, wenn bereits ein Großteil des vitalen ortständigen Knochens verloren gegangen ist. Bei der radiologischen Untersuchung zeigt sich ein Knochenverlust in der Knochenmatrix meist erst mit einer zehntägigen Verzögerung, da sich lediglich die nicht vitale, verkalkte Knochenmatrix im Röntgen- oder CT-Bild darstellen lässt und diese erst zu einem späten Zeitpunkt abgebaut wird.

Es besteht deshalb die Aufgabe, ein Osteosynthesehilfsmittel der eingangs genannten Art zu schaffen, das es ermöglicht, Fehler bei der Frakturheilung frühzeitig zu erkennen.

Diese Aufgabe wird mit den Merkmalen des Anspruchs 1 gelöst.

In vorteilhafter Weise ist es dadurch möglich, während des Heilungsprozesses auf einfache Weise die Haltekraft des mindestens einen Befestigungselements zu überprüfen, so dass für den Fall, dass sich ein oder mehrere Befestigungselemente einmal lockern sollten, rechtzeitig Gegenmaßnahmen ergriffen werden können, um einer weitergehenden Schädigung des Knochengewebes an der zu behandelnden bzw. zu synthetisierenden Stelle des Knochens vorzubeugen. Durch die Verwendung des erfindungsgemäßen Osteosynthesehilfsmittels bei Frakturen des Kieferknochens kann eine Frakturkrankheit weitestgehend vermieden werden. Selbstverständlich kann das Osteosynthesehilfsmittel aber auch zum Fixieren von Knochenfrakturen und/oder Schwächungsstellen (z.B. bei der Tumorbehandlung) anderer Knochen des menschlichen oder tierischen Körpers verwendet werden. Dabei ist es sogar möglich, dass das Verstärkungselement selbst ein Knochen Ist, dass also zwei Knochen über das Osteosynthesehilfsmittel direkt miteinander verbunden werden. Die Messeinrichtung kann über eine elektrische Leitung oder drahtlos mit der Auswerte- und/oder Anzeigeeinrichtung für die Haltekraft verbunden sein. Das Osteosynthesehilfsmittel ermöglicht sehr kompakte Abmessungen, so dass das Gewebe und der Knochen beim Implantieren des zumindest aus dem Verstärkungselements, den Befestigungselementen und dem mindestens einen Kraftsensor bestehenden Implantats geschont werden.

Bei einer zweckmäßigen Ausgestaltung der Erfindung weist der Kraftsensor mindestens eine Durchtrlitsöffnung für ein Befestigungselement auf Die Haltekraft kann dann mit großer Präzision gemessen werden. Unter einer Durchtriftsöffnung wird eine in Umfangsrichtung geschlossene Durchtrittsöffnung, wie z.B. eine o-förmige Durchtrittsöffnung, und/oder eine randoffene Durchtrittsöffnung, wie z.B. eine u-förmige Durchtrittsöffnung verstanden.

Bei einer bevorzugten Ausführungsform der Erfindung ist der Kraftsensor ein kapazitiver Krafrsensor, der mindestens zwei Elektroden aufweist die durch eine dielektrische, elastische Zwischenschicht voneinander beabstandet sind, wobei die Elektroden durch Druckbeaufschlagung entgegen der Rückstellkraft des Werkstoffs der Zwischenschicht aufeinander zu bewegbar sind, und wobei die Auswerte-und/oder Anzeigeeinrichtung zur Bestimmung der Haltekraft In Abhänglgkeit von der elektrischen Kapazität zwischen den Elektroden ausgebildet ist. Der Kraftsensor ermöglicht dann einen einfachen und kompakten Aufbau. Da die elektrische Kapazität vom Abstand der Elektroden und dieser wiederum von der Druckbeaufschlagung des Dielektrikums mit der Haltekraft des Befestigungselements abhängig ist, lässt sich durch Messung der Kapazität die Haltekraft bestimmen. Die elektrische Kapazität wird bevorzugt dadurch gemessen, dass der kapazitive Kraftsensor mit einer Induktivität einen Schwingkreis bildet, der Schwingkreis zu Eigenschwingungen angeregt und die Resonanzfrequenz des Schwingkreises gemessen wird. Aus der Resonanzfrequenz $f_{res}$ und der Induktivität L kann dann die Kapazität C wie folgt bestimmt werden:

$$C = \frac{1}{L\,(2\pi\,f_{res})^2}$$

Selbstverständlich kann die Kapazität C aber auch auf andere Weise gemessen werden, beispielsweise mit Hilfe einer Wheatstonschen Brücke.
Vorteilhaft ist, wenn die Elektroden durch eine elektrisch isolierende Mantelfolie abgedeckt und gegen die Zwischenschicht abgedichtet sind. Der Kraftsensor ist dann kostengünstig als mehrlagige Schichtanordnung herstellbar, bei der die elektrisch leitenden Elektroden vollständig im Inneren der Schichtanordnung angeordnet und somit gegen den Knochen und die diesen umgebenden Gewebeweichteile elektrisch isoliert ist. Dabei können die Elektroden ggf. als Metallisierung auf die Zwischenschicht aufgebracht sein, beispielsweise durch Bedampfen.

Bei einer anderen vorteilhaften Ausführungsform ist der Kraftsensor ein induktiver Kraftsensor, der eine Spule und ein mit dieser zusammenwirkendes, vorzugsweise plattenförmiges Joch aus einem magnetisch leitenden Werkstoff aufweist, dass die Spule und das Joch durch eine magnetisch nicht leitende elastische Zwischenschicht voneinander beabstandet und durch Druckbeaufschlagung entgegen der Rückstellkraft des Werkstoffs der Zwischenschicht aufeinander zu bewegbar sind, und dass die Auswerte- und/oder Anzeigeeinrichtung zur Bestimmung der Haltekraft in Abhängigkeit von der Induktivität der Spule ausgebildet ist. Auch dieser Kraftsensor ermöglicht einen flachen und kostengünstigen Aufbau.

Bei einer vorteilhaften Ausgestaltung der Erfindung sind an der Zwischenschicht mehrere Kraftsensoren vorgesehen, die unterschiedlichen Befestigungselementen zugeordnet und jeweils mit einem Messsignaleingang der Messeinrichtung verbunden sind. Dadurch ist es möglich, für jedes Befestigungselement des Osteosynthesehilfsmittels jeweils ein Haltekraftsignal zu messen. Bei einem Verstärkungselement, das mit einer ausreichend großen Anzahl von Befestigungselementen an dem Knochen fixiert ist, kann dann bei der Auswertung der Haltekraftsignale berücksichtigt werden, dass die Haltekraft eines Befestigungselements, das sich gelockert hat, eventuell von den übrigen Befestigungselementen übernommen wird. In diesem Fall kann das lose Befestigungselement ggf toleriert werden.

Bei einer bevorzugten Ausführungsform der Erfindung weist das Befestigungselement einen Kopfteil und einen Schaft auf, wobei die aus der Zwischenschicht, den Elektroden und ggf. den Mantelfolien und/oder der Spule und dem Joch bestehende Anordnung an dem Kopf des Befestigungselements angeordnet ist. Die auf dem Verstärkungselement befindliche Messeinrichtung kann dann direkt von dem Kopfteil beispielsweise einer Knochenschraube druckbeaufschlagt werden. Der Kraftsensor ermöglicht dann eine präzise Bestimmung der Haltekraft des Befestigungselements.

Vorteilhaft ist, wenn der kapazitive Kraftsensor und/oder der induktive Kraftsensor Teil eines LC-Schwingkreises ist, und wenn die Auswerte- und/oder Anzeigeeinrichtung einen Empfängen zur drahtlosen Detektion eines von dem LC-Schwingkreises erzeugten elektromagnetischen Felds aufweist. In dem menschlichen oder tierischen Körper brauchen dann keine aktiven elektronischen Bauteile, wie z.B. ein Halbleiterchip, implantiert zu werden. Vielmehr kann die gesamte Signalauswertung auf einer außerhalb des menschlichen oder tierischen Körpers befindlichen elektronischen Schaltung erfolgen. Gegebenenfalls ist es sogar möglich, mehrere Kraftsensoren vorzusehen, denen jeweils ein Schwingkreis zugeordnet ist, und dass die Resonanzfrequenzen dieser Schwingkreise in unterschiedlichen Frequenzbereichen liegen.

Bei einer bevorzugten Ausgestaltung der Erfindung weist die Auswerte- und/oder Anzeigeeinrichtung eine elektronische Schaltung auf, die auf einer Mantelfolie angeordnet und über Leiterbahnen mit dem mindestens einen Kraftsensor verbunden ist, und dass die elektronische Schaltung vorzugsweise mindestens ein elektronisches Bauelement aufweist, das in eine biokompatible, elektrisch isolierende Vergussmasse eingebettet ist. Die elektronische Schaltung ist also auf der Mantelfolie angeordnet und in eine Vergussmasse eingekapselt, was entsprechend kompakte Abmessungen des Implantats ermöglicht. Dabei ist es sogar möglich, dass das elektronische Bauelement ein Halbleiterchip, insbesondere ein Mikroprozessor ist. Die Vergussmasse kann ein biokompatibler Kunststoff sein, beispielsweise PDMS.

Vorteilhaft ist, wenn ein Stromversorgungsanschluss der elektronischen Schaltung über einen Gleichrichter mit einer vorzugsweise auf der Zwischenschicht angeordneten Induktivität verbunden ist, die mit einer Einrichtung zur Erzeugung eines magnetischen Wechselfelds berührungslos zusammenwirkt. Die elektronische Schaltung kann dann drahtlos über die außerhalb des menschlichen oder tierischen Körpers befindliche Einrichtung zur Erzeugung des magnetischen Wechselfelds mit Strom versorgt werden. Es sind aber auch andere Ausführungen denkbar, bei denen als Stromversorgung eine Batterie oder ein Akku vorgesehen sein.

Die elektronische Schaltung weist bevorzugt einen Datenspeicher zur Zwischenspeicherung von Messwerten auf Diese können dann zu einem späteren Zeitpunkt aus dem Datenspeicher ausgelesen und angezeigt oder beispielsweise auf einem separaten Computer ausgewertet und/oder verarbeitet werden. Dadurch ist es insbesondere möglich, über einen längeren Zeitraum den Verlauf der Haltekraft und/oder deren Änderungsgeschwindigkeit zu bestimmen.

Vorteilhaft ist, wenn die Auswerte- und/oder Anzeigeeinrichtung einen Sollwertspeicher aufweist, in dem ein Sollwertbereich für die Haltekraft abgelegt ist, und wenn der Sollwertspeicher und der Kraftsensor über eine Vergleichseinrichtung mit einer Fehlerzustandsanzeige verbunden sind. Der Patient kann dann auf einfache Weise selbst prüfen, ob die Haltekräfte der Befestigungselemente innerhalb des Sollwertbereichs liegen und beim Auftreten eines Fehlerzustands einen Arzt aufsuchen.

Nachfolgend sind Ausführungsbeispiele der Erfindung anhand der Zeichnung näher erläutert. Es zeigen:

Fig. 1 und 2     einen Querschnitt durch eine über Befestigungselemente mit einem Knochen verbundene Osteosyntheseplatte zum Fixieren einer Knochenwunde, wobei an der Osteosyntheseplatte Kraftsensoren zur Messung der Haltekrarft der Befestigungselemente angeordnet sind,

Fig. 3     einen Längsschnitt durch eine mehrere kapazitive Kraftsensoren aufweisende Messeinrichtung,

Fig. 4     eine Aufsicht auf die in Fig. 3 mit IV bezeichnete Ebene der Messeinrichtung,

Fig. 5 und 6     eine Aufsicht auf eine Messeinrichtung, bei der die elektrische Energie zum Betreiben der Kraftsensoren von einer in einem magnetischen Wechselfeld angeordneten Induktivität erzeugt wird,

Fig. 7     eine schematische Darstellung eines induktiven Kraftsensors, und

Fig. 8     eine Messeinrichtung, die einen Transponder zur drahtlosen Übertragung von Messdaten zu einem Lesegerät aufweist.

Ein chirurgisches implantat zur Wiederherstellung der Kontinuität von Knochen 1 des menschlichen oder tierischen Körpers nach einer Fraktur oder einer Osteotomie weist ein im Ganzen mit 2 bezeichnetes Osteosynthesehilfsmittel und eine Osteosyntheseplatte als Verstärkungselement 4 für den Knochen 1 auf Das Osteosynthesehilfsmütel 2 hat als Befestigungselemente 5 mehrere Knochenschrauben, die dazu vorgesehen sind, das Verstärkungselement 4 beidseits einer zu synthetisierenden Knochenstelle 3 an dem Knochen 1 zu fixieren und somit die Knochenstelle 3 gegen an dem Knochen 1 angreifende Kräfte zu entlasten. Die Befestigungselemente 5 haben jeweils einen mit dem Knochen verschraubbaren Schaft $\sigma$ und ein Kopfteil 7, welches das Verstärkungselement 4 formschlüssig hintergreift. Das Verstärkungselement 4 besteht aus einem biokompatiblen Werkstoff, beispielsweise aus Titan. Das Verstärkungselement 4 kann aber auch selbst ein Kochen oder ein Teil eines solchen sein.

Mit Hilfe des aus dem Osteosynthesehilfsmittel 2 und dem Verstärkungselement 4 gebildeten Implantats werden die beidseits der Knochenstelle 3 befindlichen Knochenbereiche relativ zueinander fixiert, so dass sich an der Knochenstelle 3 Knochen neu bilden kann. Außerdem ermöglicht das Implantat 1 bereits während des Heilungsprozesses eine mechanische Belastung des Knochens 1 und der damit verbundenen Muskulatur.

Zur Messung der Haltekräfte der einzelnen Befestigungselemente 4 ist eine Messeinrichtung vorgesehen, die für mindestens ein Befestigungselement 5 jeweils einen Kraftsensor 8 aufweist, der mit einem Messsignaleingang 28 verbunden ist. Ein Ausgang der Messeinrichtung ist an einer Auswerte- und/oder Anzeigeeinrichtung Q angeschlossen.

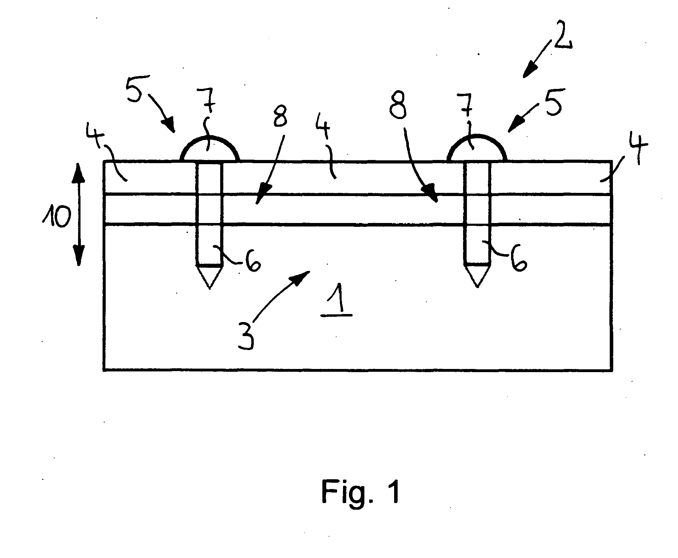

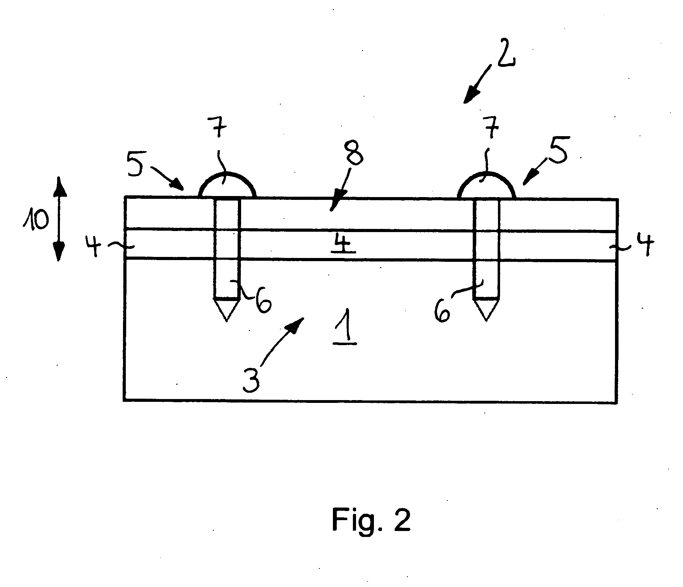

Bei an dem Knochen 1 fixiertem Implantat sind die Kraftsensoren 8 derart zwischen dem Verstärkungselement 4 und dem Knochen 1 angeordnet ist, dass sie von der Haltekraft des Befestigungselements 5 beaufschlagt werden. Bei dem in Fig. 1 gezeigten Ausführungsbeispiel sind die Kraftsensoren 8 jeweils zwischen dem Kopf 7 des ihnen zugeordneten Befestigungselements 5 und der dem Knochen 1 abgewandten Oberfläche des Verstärkungselements 4 eingespannt. Dadurch wird eine hohe Messgenauigkeit ermöglicht. Wie in Fig. 2 erkennbar ist, können die Kraftsensoren 8 aber auch zwischen dem Knochen und der diesem zugewandten Oberfläche des Verstärkungselements 4 eingespannt sein. In beiden Fällen wird etwa die gesamte Haltekraft der Befestigungselemente 5 auf die Kraftsensoren 8 ausgeübt.

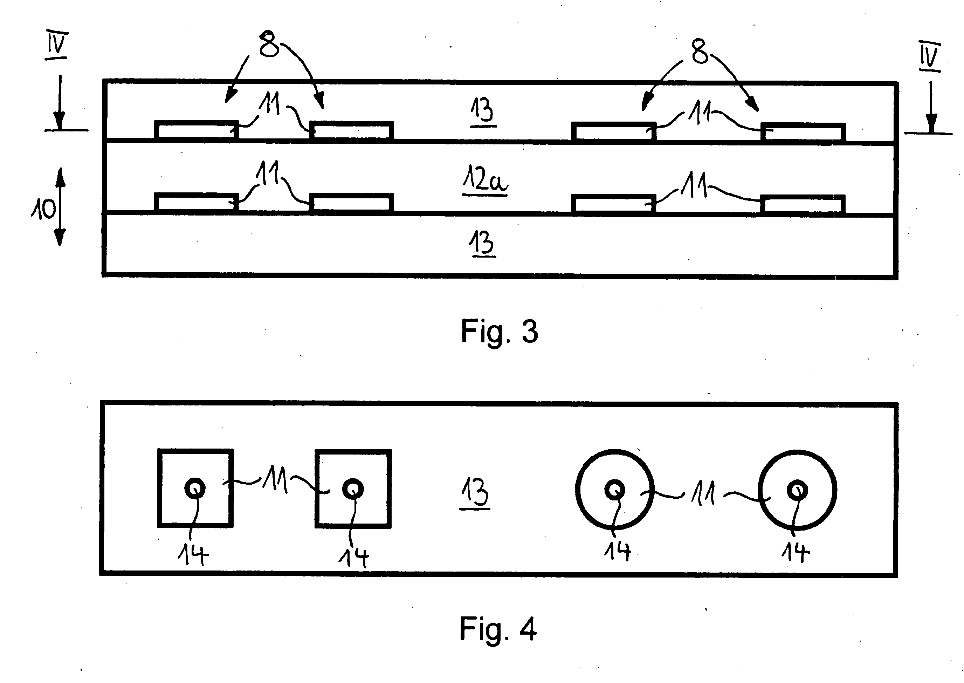

Wie in Fig. 3 und 4 besonders gut erkennbar ist, sind die Kraftsensoren 8 als flächige Elemente ausgebildet, die - wenn das Implantat mit dem Knochen verschraubt ist - etwa parallel zur Oberfläche des Knochens 1 verlaufen. Quer zu Ihren Erstreckungsebenen sind die Kraftsensoren 8 in Richtung des Doppelpfeils 10 druckempfindlich. Die einzelnen Kraftsensoren 8 haben jeweils eine Durchtrittsöffnung 14, die der Schaft 6 des Befestigungselements 5 durchsetzt.

Bei den Ausführungsbeispielen gemäß Fig. 3 bis σ sind die Kraftsensoren 8 kapazitive Kraftsensoren 8, die jeweils zwei Elektroden 11 haben, die durch eine dielektrische, elastische Zwischenschicht 12a voneinander beabstandet sind. Bei der Fertigung des Implantats können die Elektroden 11 beispielsweise durch Bedampfen auf die Zwischenschicht 12a aufgebracht werden. Die Zwischenschicht 12a kann eine Poyimidschicht oder eine PDMS-Schicht (Poly-dimethylsiloxan-Schicht) sein. Beidseits der Zwischenschicht 12a ist eine elektrisch isolierende Mantelfolie 13 vorgesehen, welche die Elektroden 11 vollständig überdeckt und welche dicht mit der Zwischenschicht 12a verbunden ist.

Durch Druckbeaufschlagung sind die Elektroden 11 entgegen der Rückstellkraft des Werkstoffs der Zwischenschicht 12a etwas aufeinander zu bewegbar. Dadurch nimmt die elektrische Kapazität des aus den Elektroden 11 und der Zwischenschicht 12a gebildeten Plattenkondensators gebildete zu. Wenn die Befestigungselemente 5 gelöst werden, nimmt die Kapazität des Plattenkondensators entsprechend ab. Die Messeinrichtung ermittelt die elektrische Kapazität zwischen den Elektroden 11 und in Abhängigkeit von dieser die Haltekraft des Befestigungselements 5.

In Fig. 4 bis 6 ist erkennbar, dass die Elektroden 11 unterschiedliche Formen aufweisen können und insbesondere ring-, rechteck- und/oder vieleckförmig ausgestaltet sein können. Dabei weist die aus den Mantelfolien 13, den Elektroden 11 und der Zwischenschicht 12a gebildete Schichtenfolge für jeden Kraftsensor 8 jeweils eine von den Elektroden 11 umgrenzte etwa kreisrunde Durchtrittsöffnung 14 auf, die der Schaft 6 durchsetzt.

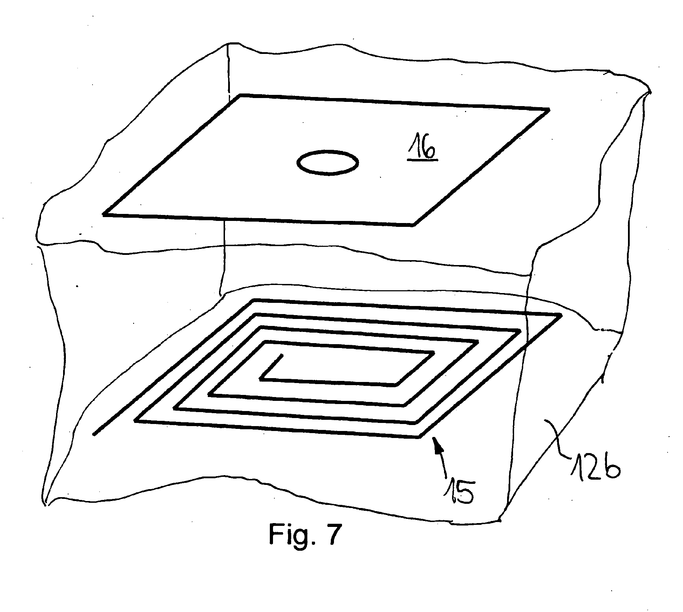

Bei dem in Fig. 7 gezeigten Ausführungsbeispiel ist der Kraftsensor 8 ein induktiver Kraftsensor 8, der eine Spule 15 und ein mit dieser zusammenwirkendes platten-oder folienförmiges Joch 16 aus einem magnetisch leitenden Werkstoff aufweist. Die Spule 15 und das Joch 16 sind durch eine magnetisch nicht leitende elastische Zwischenschicht 12b voneinander beabstandet und durch Druckbeaufschlagung entgegen der Rückstellkraft des Werkstoffs der Zwischenschicht 12b aufeinander zu bewegbar. Die Messeinrichtung ermittelt die sich dabei ändernde Induktivität der Spule 15 und bestimmt in Abhängigkeit von der Induktivität die Haltekraft des dem Kraftsensor 8 zugeordneten Befestigungselements 5. Auf der Zwischenschicht 12b können mehrere induktive Kraftsensoren 8 angeordnet sein. Ähnlich wie bei dem in Fig. 3 gezeigten Ausführungsbeispiel können auch bei einem induktiven Kraftsensor 8 beidseits der Zwischenschicht 12b Mantelfolien angeordnet sein, von denen die eine die Spule 15 und die andere das Joch 16 abdeckt. Gegebenenfalls ist es aber auch denkbar, die Mantelfolie nur auf der Seite der Zwischenschicht 12b vorzusehen, an der die Spule 15 angeordnet ist.

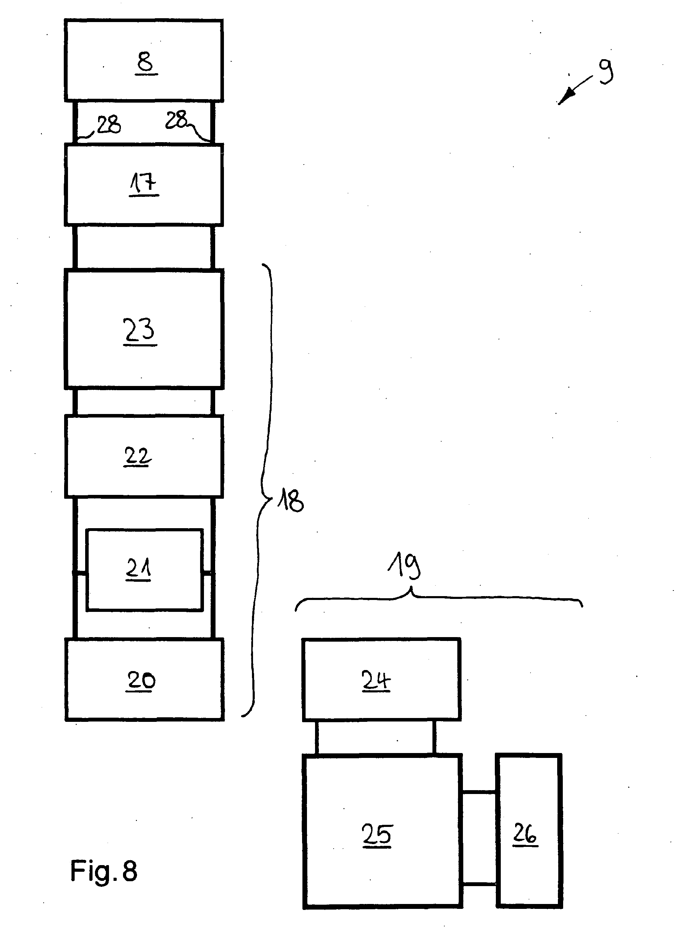

Die Auswerte- und/oder Anzeigeeinrichtung 9 weist eine elektronische Schaltung auf, die auf der Mantelfolie 13 angeordnet und über in der Zeichnung nicht näher dargestellte Leiterbahnen mit den Kraftsensoren 8 verbunden ist. Wie In Fig. 8 erkennbar ist, umfaßt die elektronische Schaltung eine mit den Kraftsensoren 8 verbundene erste Datenverarbeitungseinrichtung 17, die Messsignaleingänge 28 hat, die an den Kraftsensoren 8 angeschlossen sind. Die Datenverarbeitungseinrichtung 17 hat einen in der Zeichnung nicht näher dargestellten Datenspeicher zur Zwischenspeicherung von Messwerten. Die Datenverarbeitungseinrichtung 17 kann ferner einen Filter zur Filterung der Messsignale der Kraftsensoren 8 aufweisen.

In Fig. 8 ist erkennbar, dass die Datenverarbeitungseinrichtung 17 an einem an sich bekannten Transponder 18 angeschlossen ist, der einerseits zur Stromversorgung der Datenverarbeitungseinrichtung 17 und andererseits zur drahtlosen Übertragung der von der Datenverarbeitungseinrichtung 17 bereitgestellten Messwerte zu einem außerhalb des menschlichen oder tierischen Körpers abgeordneten Lesegerät 19 dient. Der Transponder 18 weist einen ersten LC-Schwingkreis 20 auf, der über eine erste Modulationseinrichtung 21, einen Gleichrichter 22 und eine Spannungsstabilisierungseinrichtung 23 mit der Datenverarbeitungseinrichtung 17 verbunden ist.

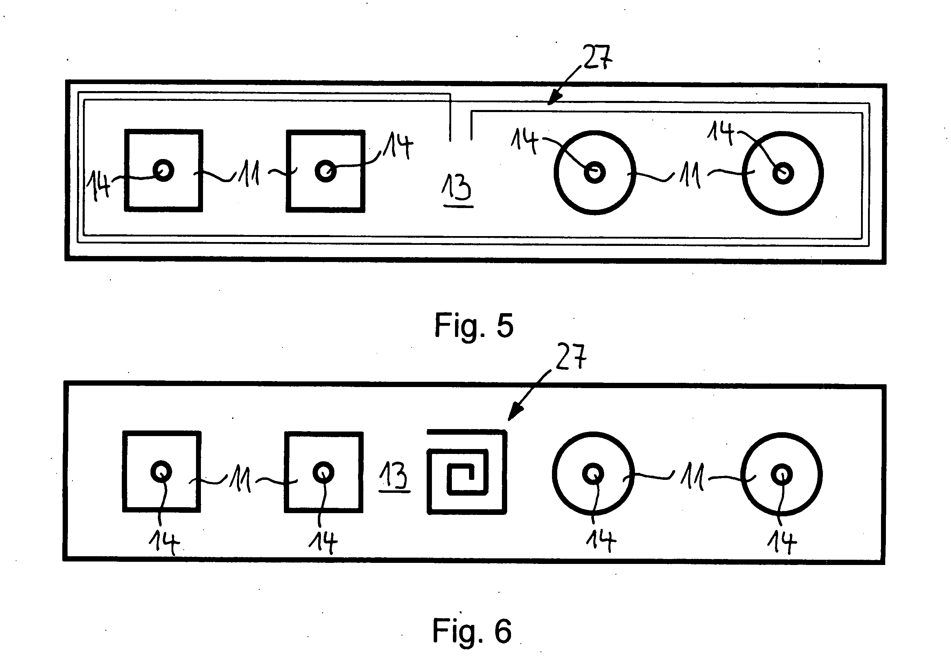

Das Lesegerät 19 weist einen zweiten LC-Schwingkreis 24 auf, der mit einer zweiten Datenverarbeitungseinrichtung 25 verbunden ist, an der eine Anzeige 26 angeschlossen ist. Mit Hilfe der zweiten Datenverarbeitungseinrichtung 25 können in dem zweiten LC-Schwingkreis 24 elektrische Schwingungen angeregt werden, die ein elektromagnetisches Feld erzeugen, das in dem ersten LC-Schwingkreis 20 eine elektrische Spannung induziert, aus der mittels des Gleichrichters eine Betriebsspannung für die erste Datenverarbeitungseinrichtung 17 und die Kraftsensoren 8 gewonnen wird. In Fig. 5 und 6 ist erkennbar, dass der erste LC-Schwingkreis 20 eine Induktivität 27 aufweist, die durch auf der Zwischenschicht 12a, 12b angeordnete Leiterbahnen gebildet ist. Die dem Ausführungsbeispiel gemäß Fig. 5 sind die Kraftsensoren 8 innerhalb des von der Induktivität 27 umgrenzten Bereichs angeordnet. Die Induktivität 27 kann aber auch zwischen den Kraftsensoren 8 angeordnet sein, wie in Fig. 6 erkennbar ist.

Mit Hilfe der Modulationseinrichtung 21 kann die Dämpfung des ersten LC-Schwingkreises 20 in Abhängigkeit von den von der Datenverarbeitungseinrichtung 17 bereitgestellten Messwerten verändert werden. Das so erhaltene Modulationssignal wird mittels des zweiten LC-Schwingkreis 24 detektiert, um die Messwerte an die zweite Datenverarbeitungseinrichtung 25 zu übermitteln.

Dort werden Sie mit einem Sollwertbereich für die Haltekräfte verglichen. Das Ergebnis des Vergleichs wird mit Hilfe der Anzeige ausgegeben. Selbstverständlich ist es aber auch denkbar, die Messwerte selbst über die Anzeige auszugeben.

**Patentansprüche**

1. Osteosynthesehllfsmittel (2) zur Wiederherstellung der Kontinuität von Knochen (1) des menschlichen oder tierischen Körpers nach einer Fraktur oder einer Osteotomie, umfassend

   - ein am Knochen (1) anbringbares Verstärkungselement (4), wie z.B. eine Osteosyntheseplatte,
   - mindestens ein Befestigungselement (5), wie z.B. einer Knochenschraube und/oder ein Knochennagel, zum Verbinden des Verstärkungselements (4) mit dem Knochen, und
   - eine mit einer Auswerte- und/oder Anzeigeeinrichtung (9) verbundene Messeinrichtung, die mindestens einen Kraftsensor (8) aufweist, der als flächiges, quer zu seiner Erstreckungsebene druckempfindliches Element ausgebildet ist,

   **dadurch gekennzeichnet,**
   **dass** der Kraftsensor (8) zur Messung der Haltekraft des zumindest einen Befestigungselements (5) derart zwischen dem zumindest einen Befestigungselement (5) und dem Verstärkungselement (4) und/oder dem Knochen (1) anbringbar ist, dass der Kraftsensor (8) von der Haltekraft des Befestigungselements (5) beaufschlagt wird.

2. Osteosynthesehilfsmittel (2) nach Anspruch 1, **da-**

durch gekennzeichnet, dass** der Kraftsensor (8) mindestens eine Durchtrittsöffnung (14) für ein Befestigungselement (5) aufweist.

3. Osteosynthesehilfsmittel (2) nach Anspruch 1 oder 2, **dadurch gekennzeichnet, dass** der Kraftsensor (8) ein kapazitiver Kraftsensor (8) ist, der mindestens zwei Elektroden (11) aufweist, die durch eine dielektrische, elastische Zwischenschicht (12a) voneinander beabstandet sind, dass die Elektroden (11) durch Druckbeaufschlagung entgegen der Rückstellkraft des Werkstoffs der Zwischenschicht (12a) aufeinander zu bewegbar sind, und dass die Auswerte- und/oder Anzeigeeinrichtung (9) zur Bestimmung der Haltekraft in Abhänglgkeit von der elektrischen Kapazität zwischen den Elektroden (11) ausgebildet ist.

4. Osteosynthesehilfsmittel (2) nach Anspruch 3, **dadurch gekennzeichnet, dass** die Elektroden (11) durch eine elektrisch isolierende Mantelfolie (13) abgedeckt und gegen die Zwischenschicht (12a) abgedichtet sind.

5. Osteosynthesehilfsmittel (2) nach Anspruch 3 oder 4, **dadurch gekennzeichnet, dass** an der Zwischenschicht (12a) mehrere Kraftsensoren (8) vorgesehen sind, die unterschiedlichen Befestigungselementen (5) zugeordnet und jeweils mit einem Messsignaleingang (28) der Messeinrichtung verbunden sind.

6. Osteosynthesehilfsmittel (2) nach einem der Ansprüche 1 bis 5, **dadurch gekennzeichnet, dass** der Kraftsensor (8) ein induktiver Kraftsensor (8) ist, der eine Spule (15) und ein mit dieser zusammenwirkendes, vorzugsweise plattenförmiges Joch (16) aus einem magnetisch leitenden Werkstoff aufweist, dass die Spule (15) und das Joch (16) durch eine magnetisch nicht teitende elastische Zwischenschicht (12b) voneinander beabstandet und durch Druckbeaufschlagung entgegen der Rückstellkraft des Werkstoffs der Zwischenschicht (12b) aufeinander zu bewegbar sind, und dass die Auswerte-und/oder Anzeigeeinrichtung (9) zur Bestimmung der Haltekraft in Abhängigkeit von der Induktivität der Spule (15) ausgebildet ist.

7. Osteosynthesehilfsmittel (2) nach einem der Ansprüche 3 bis 6, **dadurch gekennzeichnet, dass** das mindestens eine Befestigungselement einen Kopfteil und einen Schaft aufweist, wobei die aus der Zwischenschicht (12a, 12b), den Elektroden (11) und ggf den Mantelfolien (1 3) und/oder der Spule (15) und dem Joch (16) bestehende Anordnung an dem Kopf des Befestigungselements angeordnet ist.

8. Osteosynthesehilfsmittel (2) nach einem der Ansprüche 3 bis 7, **dadurch gekennzeichnet, dass** der kapazitive Kraftsensor (8) und/oder der induktive Kraftsensor (8) Teil eines LC-Schwingkreises ist, und dass die Auswerte-und/oder Anzeigeeinrichtung (9) einen Empfänger zur drahtlosen Detektion eines von dem LC-Schwingkreises erzeugten elektromagnetischen Felds aufweist.

9. Osteosynthesehilfsmittel (2) nach einem der Ansprüche 1 bis 8, **dadurch gekennzeichnet, dass** die Auswerte- und/oder Anzeigeeinrichtung (9) eine elektronische Schaltung aufweist, die auf einer Mantelfolie (1 3) angeordnet und über Leiterbahnen mit dem mindestens einen Kraftsensor (8) verbunden ist, und dass die elektronische Schaltung vorzugsweise mindestens ein elektronisches Bauelement aufweist, das In eine biokompatible, elektrisch isolierende Vergussmasse eingebettet ist.

10. Osteosynthesehilfsmittel (2) nach Anspruch 9, **dadurch gekennzeichnet dass** ein Stromversorgungsanschluss der elektronischen Schaltung über einen Gleichrichter (22) mit einer vorzugsweise auf der Zwischenschicht angeordneten Induktivität (27) verbunden ist, die mit einer Einrichtung zur Erzeugung eines magnetischen Wechselfelds berührungslos zusammenwirkt.

11. Osteosynthesehilfsmittel (2) nach Anspruch 9 oder 10, **dadurch gekennzeichnet dass** die elektronische Schaltung einen Datenspeicher zur Zwischenspeicherung von Messwerten aufweist.

12. Osteosynthesehilfsmittel (2) nach einem der Ansprüche 1 bis 11, **dadurch gekennzeichnet, dass** die Auswerte- und/oder Anzeigeeinrichtung (9) einen Sollwertspeicher aufweist, in dem ein Sollwertbereich für die Haltekraft abgelegt ist, und dass der Sollwerbpeicher und der Kraftsensor (8) über eine Vergleichseinrichtung mit einer Fehlerzustandsanzeige verbunden sind.

**Claims**

1. Osteosynthesis aid (2) for restoring the continuity of bones (1) in the human or animal body following a fracture or an osteotomy, comprising

- a strengthening element (4), such as e.g. an osteosynthesis plate, which can be attached to the bone (1),
- at least one attachment element (5), such as e.g. a bone screw and/or a bone nail, for connecting the strengthening element (4) to the bone, and
- a measuring apparatus, which is connected to an evaluation and/or display apparatus (9) and has at least one force sensor (8) which is embodied as a two-dimensional element that is sensitive to pressure perpendicular to the plane of extent thereof,

**characterized**
**in that** the force sensor (8), for the purposes of measuring the retention force of the at least one attachment element (5), can be attached between the at least one attachment element (5) and the strengthening element (4) and/or the bone (1) in such a way that the retention force of the attachment element (5) acts upon the force sensor (8).

2. Osteosynthesis aid (2) according to Claim 1, **characterized in that** the force sensor (8) has at least one passage opening (14) for an attachment element (5).

3. Osteosynthesis aid (2) according to Claim 1 or 2, **characterized in that** the force sensor (8) is a capacitive force sensor (8), which has at least two electrodes (11) spaced apart by a dielectric, elastic intermediate layer (12a), **in that** the electrodes (11) are moveable towards one another against the restoration force of the material of the intermediate layer (12a) by the application of pressure, and **in that** the evaluation and/or display apparatus (9) is configured to determine the holding force as a function of the electrical capacitance between the electrodes (11).

4. Osteosynthesis aid (2) according to Claim 3, **characterized in that** the electrodes (11) are covered by an electrically insulating casing film (13) and sealed against the intermediate layer (12a).

5. Osteosynthesis aid (2) according to Claim 3 or 4, **characterized in that** a plurality of force sensors (8) are provided on the intermediate layer (12a), which force sensors are associated with different attachment elements (5) and respectively connected to a measurement signal input (28) of the measuring apparatus.

6. Osteosynthesis aid (2) according to one of Claims 1 to 5, **characterized in that** the force sensor (8) is an inductive force sensor (8), which has a coil (15) and, interacting therewith, a preferably plate-shaped yoke (16) made of a magnetically conductive material, **in that** the coil (15) and the yoke (16) are separated from one another by a magnetically non-conductive elastic intermediate layer (12b) and are moveable towards one another against the restoration force of the material of the intermediate layer (12b) by the application of pressure, and **in that** the evaluation and/or display apparatus (9) is configured to determine the holding force as a function of the inductance of the coil (15).

**7.** Osteosynthesis aid (2) according to one of Claims 3 to 6, **characterized in that** the at least one attachment element has a head part and a shaft, wherein the arrangement consisting of the intermediate layer (12a, 12b), the electrodes (11) and optionally the casing films (13) and/or the coil (15) and the yoke (16) is arranged on the head of the attachment element.

**8.** Osteosynthesis aid (2) according to one of Claims 3 to 7, **characterized in that** the capacitive force sensor (8) and/or the inductive force sensor (8) is part of an LC resonant circuit and **in that** the evaluation and/or display apparatus (9) has a receiver for wireless detection of an electromagnetic field generated by the LC resonant circuit.

**9.** Osteosynthesis aid (2) according to one of Claims 1 to 8, **characterized in that** the evaluation and/or display apparatus (9) has an electronic circuit which is arranged on a casing film (13) and connected to the at least one force sensor (8) by conductor tracks and **in that** the electronic circuit preferably has at least one electronic component, which is embedded in a biocompatible, electrically insulating casting compound.

**10.** Osteosynthesis aid (2) according to Claim 9, **characterized in that** a power supply connector of the electronic circuit is connected to an inductor (27), which is preferably arranged on the intermediate layer, via a rectifier (22), which inductor interacts contactlessly with an apparatus for generating an alternating magnetic field.

**11.** Osteosynthesis aid (2) according to Claim 9 or 10, **characterized in that** the electronic circuit comprises a data storage medium for buffer storing measured values.

**12.** Osteosynthesis aid (2) according to one of Claims 1 to 11, **characterized in that** the evaluation and/or display apparatus (9) has an intended value storage medium, in which an intended value range for the holding force is stored, and **in that** the intended value storage medium and the force sensor (8) are connected via a comparison apparatus to an error-state display.

**Revendications**

**1.** Accessoire d'ostéosynthèse (2) permettant de préserver la continuité de l'os (1) du corps humain ou animal après une fracture ou une ostéotomie, l'accessoire comprenant :

un élément de renfort (4), par exemple une plaque d'ostéosynthèse, apte à être placé sur l'os (1),

au moins un élément de fixation (5), par exemple une vis à os et/ou un clou à os, destiné à relier l'élément de renfort (4) et l'os et

un dispositif de mesure relié à un dispositif d'évaluation et/ou d'affichage (9) et présentant au moins une sonde de force (8) configurée comme élément plat sensible à la pression dans la direction transversale par rapport au plan de son extension, **caractérisé en ce que**

la sonde de force (8) peut être placée entre le ou les éléments de fixation (5), l'élément de renfort (4) et/ou l'os (1) pour mesurer la force de maintien du ou des éléments de fixation (5) de telle sorte que la force de maintien de l'élément de fixation (5) puisse être appliquée sur la sonde de force (8).

**2.** Accessoire d'ostéosynthèse (2) selon la revendication 1, **caractérisé en ce que** la sonde de force (8) présente au moins une ouverture (14) permettant le passage d'un élément de fixation (5).

**3.** Accessoire d'ostéosynthèse (2) selon les revendications 1 ou 2, **caractérisé en ce que** la sonde de force (8) est une sonde de force (8) capacitive qui présente au moins deux électrodes (11) maintenues à distance l'une de l'autre par une couche intermédiaire élastique diélectrique (12a), **en ce que** les électrodes (11) peuvent être déplacées en direction l'une de l'autre par application d'une poussée en opposition à la force de rappel du matériau de la couche intermédiaire (12a) et **en ce que** le dispositif d'évaluation et/ou d'affichage (9) est configuré pour déterminer la force de maintien en fonction de la capacité électrique qui règne entre les électrodes (11).

**4.** Accessoire d'ostéosynthèse (2) selon la revendication 3, **caractérisé en ce que** les électrodes (11) sont recouvertes par une feuille d'enveloppe (13) électriquement isolante et sont séparées hermétiquement de la couche intermédiaire (12a).

**5.** Accessoire d'ostéosynthèse (2) selon les revendications 3 ou 4, **caractérisé en ce que** plusieurs sondes de force (8) associées à différents éléments de fixation (5) et reliées à des entrées (28) de signal de mesure respectives du dispositif de mesure sont prévues sur la couche intermédiaire (12a).

**6.** Accessoire d'ostéosynthèse (2) selon l'une des revendications 1 à 5, **caractérisé en ce que** la sonde de force (8) est une sonde de force (8) inductive qui présente une bobine (15) et une culasse (16) en un matériau magnétiquement conducteur, de préférence en forme de plaque et coopérant avec la bobine, **en ce que** la bobine (15) et la culasse (16) sont maintenues à distance l'une de l'autre par une couche intermédiaire (12b) élastique et magnétiquement non conductrice et peuvent être déplacées en direction l'une de l'autre par l'application d'une poussée en opposition à la force de rappel du matériau de la couche intermédiaire (12b) et **en ce que** le dispositif d'évaluation et/ou d'affichage (9) est configuré pour déterminer la force de maintien en fonction de l'inductance de la bobine (15).

7. Accessoire d'ostéosynthèse (2) selon l'une des revendications 3 à 6, **caractérisé en ce que** le ou les éléments de fixation présentent une partie de tête et une tige, l'ensemble constitué de la couche intermédiaire (12a, 12b), des électrodes (11) et éventuellement de la feuille d'enveloppe (13) et/ou de la bobine (15) et de la culasse (16) étant disposé sur la tête de l'élément de fixation.

8. Accessoire d'ostéosynthèse (2) selon l'une des revendications 3 à 7, **caractérisé en ce que** la sonde de force (8) capacitive et/ou la sonde de force (8) inductive font partie d'un circuit oscillant LC et **en ce que** le dispositif d'évaluation et/ou d'affichage (9) présente un récepteur qui permet une détection sans fil du champ électromagnétique produit par le circuit oscillant LC.

9. Accessoire d'ostéosynthèse (2) selon l'une des revendications 1 à 8, **caractérisé en ce que** le dispositif d'évaluation et/ou d'affichage (9) présentent un circuit électronique disposé sur une feuille d'enveloppe (13) et relié par des pistes conductrices à la sonde ou aux sondes de force (8) et **en ce que** le circuit électronique présente de préférence au moins un composant électronique incorporé dans une pâte de scellement biocompatible et électriquement isolante.

10. Accessoire d'ostéosynthèse (2) selon la revendication 9, **caractérisé en ce qu'**une borne d'alimentation en courant du circuit électronique est reliée par l'intermédiaire d'un redresseur (22) à une inductance (27) disposée de préférence sur la couche intermédiaire et qui coopère sans contact avec un dispositif de production d'un champ magnétique alternatif.

11. Accessoire d'ostéosynthèse (2) selon l'une des revendications 9 ou 10, **caractérisé en ce que** le circuit électronique présente une mémoire de données qui conserve temporairement des valeurs de mesure.

12. Accessoire d'ostéosynthèse (2) selon l'une des revendications 1 à 11, **caractérisé en ce que** le dispositif d'évaluation et/ou d'affichage (9) présentent une mémoire à valeurs de consigne dans laquelle une plage de valeurs de consigne de la force de maintien est conservée et **en ce que** la mémoire à valeurs de consigne et la sonde de force (8) sont reliées à un affichage d'état de défaut par l'intermédiaire d'un dispositif comparatif.

Fig. 1

Fig. 2

Fig. 3

Fig. 4

Fig. 5

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20040204647 A1
- WO 2005074821 A2
- DE 9004717 U1